Europäisches Patentamt

European Patent Office

Office européen des brevets

Publication number: **0 288 793**
**A2**

# EUROPEAN PATENT APPLICATION

(21) Application number: 88105533.9

(22) Date of filing: 07.04.88

(51) Int. Cl.⁴: **B01L 3/00 , G01N 33/50 ,**
**//G01N35/02,G01N33/574,**
**G01N33/76,G01N33/68,**
**G01N33/543**

(30) Priority: 22.04.87 US 41190

(43) Date of publication of application:
**02.11.88 Bulletin 88/44**

(84) Designated Contracting States:
**AT BE CH DE ES FR GB IT LI NL**

(71) Applicant: **ABBOTT LABORATORIES**

**Abbott Park Illinois 60064(US)**

(72) Inventor: **Brown, William E. III**
**17391 West Woodland Drive**
**Grayslake Illinois 60030(US)**
Inventor: **Sebesta, Edward E.**
**311 West Sheridan Place**
**Lake Bluff Illinois 60044(US)**
Inventor: **Osikowicz, Eugene W.**
**24107 West Marydale**
**Lake Zurich Illinois 60647(US)**
Inventor: **Ramp, Sally K.**
**462 Pine Grove**
**Gurnee Illinois 60031(US)**
Inventor: **Grandone, Cass J.**
**494 N. Oakwood Avenue**
**Lake Forest Illinois 60045(US)**

(74) Representative: **Modiano, Guido et al**
**MODIANO, JOSIF, PISANTY & STAUB**
**Modiano & Associati Baaderstrasse 3**
**D-8000 München 5(DE)**

(54) **Cartridge and methods for performing a solid-phase immunoassay.**

(57) A disposable cartridge suitable for performing automated solid-phase diagnostic assays which employs microparticles to complex an analyte and where the microparticle complex becomes retained and immobilized on a fibrous matrix such that the presence of analyte on the microparticles can be detected by optical means. A cartridge is disclosed which comprises a plurality of wells at least one of the wells comprises an entrance port and holding means for receiving and holding a quantity of sample and assay reagents, a fibrous matrix for retaining and immobilizing microparticle/analyte complexes for detection, the fibrous matrix positioned below the holding means, and having an average spatial separation of fibers greater than the average diameter of said microparticles, and an absorbent material positioned below the fibrous matrix to absorb any sample or assay reagents that flow through said fibrous matrix.

FIG. 2

## CARTRIDGE AND METHODS FOR PERFORMING A SOLID-PHASE IMMUNOASSAY

### Background of the Invention

The present invention is directed toward a disposable cartridge suitable for use in an automated solid-phase immunoassay. The cartridge is designed to have a plurality of wells where a sample material can be treated with reagents to perform a solid-phase assay and in which the results of that assay can be optically read.

Techniques for performing an immunoassay are generally well known in the art. For example, conventional enzyme immunoassay procedures involve a series of steps wherein an analyte in a sample material is initially bound to a corresponding antigen or antibody reagent. A second antigen or antibody is then introduced into the sample which has been labeled or conjugated with an enzyme or other substance capable of detection when treated with an additional suitable indicator reagent such as a chromogen or dye to produce a signal which is then read to indicate the absence or presence of the antigen or antibody in the sample.

Solid-phase immunoassay procedures are preferred over other diagnostic methods because of their safety, ease of use, specificity and sensitivity. Moreover, when employing a solid-phase immunoassay procedure, the result is easily observed by instruments which increases the accuracy of the procedure.

One form of a conventional solid-phase immunoassay is a "sandwich assay" which involves contacting a test sample suspected of containing an antibody or antigen with a substantially solid inert plastic or glass bead or other support material which can be coated with a protein or another substance capable of binding the antigen or antibody to the surface of the support. After the antibody or antigen is bound to the support material it is treated with a second antigen or antibody, which is conjugated with an enzyme. The second antigen or antibody then becomes bound to the corresponding antibody or antigen on the support. Following one or more washing steps to remove any unbound material an indicator substance, for example, a chromogenic substance, is added which reacts with the enzyme to produce a color change. The color change can be observed visually or more preferably by an instrument to indicate the presence or absence of an antibody or antigen in the sample.

Many procedures and apparatus have been designed to perform solid-phase immunoassays. U.S. Patent 4,632,901 discloses an apparatus having a porous filter containing a bound receptor for complexing an analyte. In this apparatus an absorbent material is positioned below the porous filter to assist the fluid sample in flowing through the filter. A labeled antibody is then added to the porous filter to detect the presence or absence of the analyte.

In another approach, European Patent Application No. 0131934 discloses an assay cartridge having a plurality of aligned adjacent reaction wells located on its top surface which empty through a filter membrane located above a waste reservoir. An EIA reaction mixture is placed in the well and drawn through the membrane by applying reduced pressure to the waste reservoir to separate a solid-phase reaction product from a liquid-phase reaction product so that the solid-phase reaction product can be observed.

Other methods for performing a solid-phase enzyme immunoassay are disclosed in U.S. Patents 4,587,102, and 4,552,839, and European Patent Application 0200381. These references generally disclose procedures for employing particles having a receptor to bind an analyte which is subsequently labeled and deposited on a matrix or other support system. The particle complex is treated with an indicator substance to indicate the presence or absence of an analyte.

While many immunoassay procedures and devices have proved useful, better procedures and devices are continually being sought to improve reliability, efficiency and sensitivity. The present invention provides all these improvements.

### Summary of the Invention

In one aspect, the present invention is directed toward a cartridge suitable for performing an automated solid-phase diagnostic assay. The ability to perform an automated assay contributes to increased reliability and efficiency. An automated system also provides a method whereby the generation of an assay signal can be rapidly read as it develops to provide a rate measurement. The ability to measure the rate of color or signal development greatly enhances sensitivity because it is self-calibrating and enables one to quantify the analyte assayed.

The cartridge comprises a sample well for receiving a sample material and a reaction well for retaining and immobilizing a microparticle, analyte complex. In a preferred embodiment of the cartridge, there are a plurality of wells for receiving reaction mixtures prior to their being added to the reaction well.

The reaction well has an entrance port and a means for holding a quantity of sample and assay reaction mixtures positioned over a fibrous matrix which retains and immobilizes microparticle analyte complexes. The fibrous matrix is composed of fibers having an average spatial separation greater than the average diameter of the microparticles. Preferably the average fiber spatial separation is greater than 10 microns.

The reaction well further comprises an absorbent material positioned below the fibrous matrix to enhance the flow of sample and assay reaction mixtures through the fibrous matrix. Generally, the absorbent material is a fibrous material whose fibers predominantly lie in a plane perpendicular to the lower surface of the fibrous matrix. The reaction well preferably contains positioning means for securely holding the absorbent material in contact with the lower surface of the fibrous matrix. Generally, this can include fins extending from the inner sides of the reaction well or a spike located at the bottom of the reaction well which force the absorbent material up, and in contact with, the lower surface of the fibrous matrix.

The present invention also provides various methods for performing a solid-phase assay in the cartridge. One method for performing a sandwich immunoassay in accordance with the invention comprises the following steps:

a) incubating the sample material with an analyte specific conjugate in a well other than the reaction well to form an analyte/conjugate complex;

b) adding microparticles having an average diameter smaller than the average spatial separation of the fibers forming the fibrous matrix to the reaction well and then transferring the analyte/conjugate complex to the reaction well to form a microparticle, analyte/conjugate complex;

c) adding a wash solution to the reaction well to transfer the microparticle analyte/conjugate complex into the fibrous matrix where it is retained and immobilized;

d) adding a substance capable of producing a signal in the presence of the microparticle analyte/conjugate complex to the fibrous matrix; and

e) detecting the signal produced.

Alternatively, steps a) can be performed by simultaneously adding the microparticle, sample and analyte specific conjugate in a well other than the reaction well and then transferring the mixture to the reaction well. In another alternative method, step b) can be performed by contacting the analyte/conjugate complex with microparticles in a well other than the reaction well to form a microparticle analyte/conjugate complex and then transferring this complex to the reaction well.

In another embodiment of the present invention, a solid-phase assay can be performed in the cartridge as described above by replacing steps a) and b) with the following steps:

a) adding a sample and microparticles having an average diameter smaller than the average spatial separation of the fibers forming the fibrous matrix to the reaction well, and incubating to form a microparticle analyte complex;

b) adding an analyte specific conjugate to the reaction well and incubating to form a microparticle analyte/conjugate complex;

In yet another embodiment step a) can be performed by incubating a sample with the microparticles in a well other than the reaction well and then adding the microparticle analyte complex formed to the reaction well. Alternatively, step b) can be performed by incubating a microparticle analyte complex with an analyte specific conjugate to form a microparticle analyte/conjugate complex, which is transferred to the reaction well.

The disposable cartridge can also be employed to conduct competitive assays. A competitive assay comprises incubating a sample and microparticles with an enzyme labeled antigen in the reaction well, adding a wash to the reaction well, adding a substrate the reaction well and detecting the product formed.

## Brief Description of the Drawings

Fig. 1 is a top plane view for one embodiment of the diagnostic cartridge.

Fig. 2 is a side view and partial cross-section of the diagnostic cartridge of Fig 1.

Fig. 3 is a graph of rapid readings conducted on a fluorescence immunoassay to determine the rate of flourescence generation employing a diagnostic cartridge and method of the present invention.

3

Fig. 4 is a perspective view of an automated analyzer suitable for use with the diagnostic cartridge. The front cover is removed to expose the functional components.

## Detailed Description of the Invention

The present invention is directed toward a cartridge and methods for performing a solid-phase immunoassay with the cartridge. The cartridge is disposable and is suitable for use with an apparatus having programmed instructions and means for transferring and reacting assay reagents and optically reading the results of the assay. The present invention also provides various methods for performing a solid-phase assay employing the disposable cartridge.

The cartridge is designed to be employed in a variety of solid-phase diagnostic assays such as sandwich or competitive binding assays. Further the cartridge is designed so that it can used for fluorescence, chemilumenescence, or colorimetric detection methods.

The cartridge is molded to have at least two wells, a sample well for receiving a sample and a reaction well for carrying out the solid-phase assay procedure; however, any number of separate wells for performing various reactions associated with a solid-phase diagnostic assay can be employed. Preferably the cartridge is molded of styrene, polycarbonate or some other moldable material which is substantially inert to the assay procedure.

Samples which can be assayed by the cartridge include biological fluids such as whole blood, spinal fluid, urine, serum, and plasma. It is also envisioned that other fluid samples of a nonbiological nature can be analyzed using the disposable cartridge. The sample is placed either manually or mechanically into the sample well and processed. The sample well can be any of the wells present on the cartridge except for the reaction well which is discussed below. Thus, the exact position of the sample well is not critical.

In a preferred embodiment the cartridge is placed in a cartridge tray or carousel designed to hold a plurality of cartridges for use in an automated analyzer. The carousel can be moved in such a manner that robotic means having multiple degrees of freedom can access each of the wells. The robotic means are equipped with probes, pipettes or other transfer devices to aspirate, add, mix or transfer sample, reagents or other necessary substances to perform a solid-phase assay in the cartridge wells.

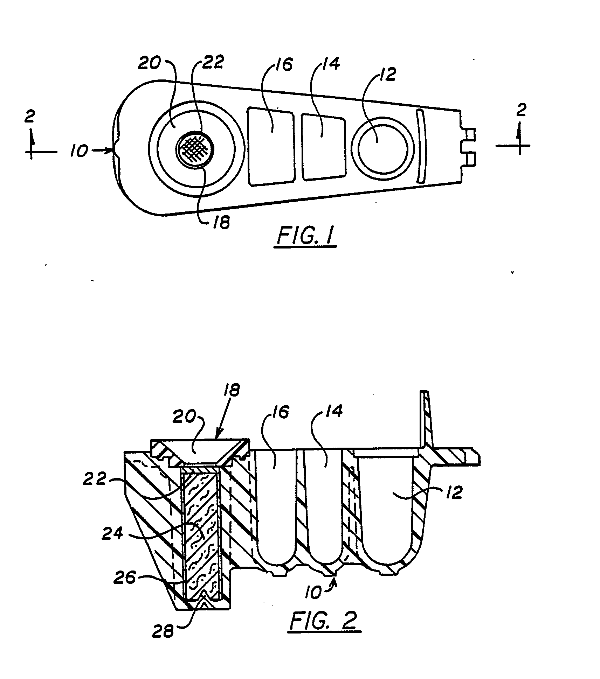

In a preferred embodiment, the cartridge 10 is shown in Figures 1 and 2. The separate wells as shown consist of three empty wells 12, 14, and 16 and a well 18 having fibrous and absorbent materials positioned therein. The latter well 18 is referred to as the "reaction well". The reaction well 18 is preferably located at the outer most position on the cartridge so that when it is placed in the carousel it can be readily scanned by an optical means to read the formation of an assay signal. After the assay is performed the cartridge can be removed from the carousel and disposed.

The extra empty wells which may be present in any one particular cartridge may be used for reacting or incubating a sample with reagents necessary for performing a solid-phase assay. While any of the wells can be designated a "sample well" it is preferred to employ the innermost well 12 as shown in Figs. 1 and 2 as the sample well. This is to simplify the transferring of sample in a sequential manner from the inner region of the cartridge to the outer region of the cartridge by automated means.

A main and essential characteristic of the cartridge is that at least one of the plurality of wells has a fibrous matrix for retaining and immobilizing microparticles for use in a solid-phase immunoassay procedure. This particular well is termed the "reaction well". Preferably the reaction well 18 as shown in Figs. 1 and 2 is located at the outermost region of the cartridge. Thus, when the cartridge is positioned in a carousel the reaction well 18 will be located at the outer circumference. This facilitates the reading of an assay signal by an optics system which may be the of an automated instrument.

The reaction well generally comprises an entrance port and holding means 20, a porous fibrous matrix 22, and an absorbent material 24. The entrance port and holding means 20 can be a molded portion of the cartridge or preferably a funnel-like structure which is press-fitted or sonic welded into the orifice of the reaction well 18. The holding means 20 is designed with sloping sides which contact the upper surface of the fibrous matrix 22 and is sized to hold a sufficient amount of sample, conjugate material or other reagents to satisfy the requirements of the particular assay being performed. While the holding means 20 can be any color a darker color or preferably black may be chosen to decrease background interference with the optics system.

The porous fibrous matrix 22 is a thin, disk like material positioned below the entrance port and holding means 20 to retain and immobilize a microparticle complex from which an assay signal can be read. The phrase "retain and immobilize" means that the microparticles, while upon the fibers of the material, are not capable of substantial movement to positions elsewhere within the material, (i.e., to other fibers), and cannot be removed completely from the material without destroying the material.

The pore size or spatial separation of the fibers comprising the fibrous matrix 22 is essential to the overall performance of the solid-phase immunoassay contemplated by the present invention. Specifically, the spatial separation of the fibers must be larger than the diameter of the microparticles employed in the assay. This is to assure that even after the microparticles are immobilized on the fibers adequate void areas will exist to assure the proper flow of reagents and sample through the fibrous matrix. Despite the large spatial separation of the fibers, the microparticles do not pass through the fibrous matrix; instead, the microparticles are immobilized upon the fibers themselves. The immobilization is not by entrapment, filtration or covalent bonding. The exact interaction between the microparticles and the fibers is not completely understood; however, the interaction that does result has been found to be especially appropriate for the performance of the subject solid-phase immunoassay methods. That is, after the microparticles are deposited on the fibrous matrix, the matrix is not blocked but instead remains porous. As used herein "porous" means that the matrix is and remains a material into which fluids can flow and can easily pass through.

The fibrous material of the present invention can be chosen from any of a variety of materials such as glass, cellulose, nylon or other natural or synthetic fibrous material well known to those skilled in the art. A preferred material is HTMV Product No. HC4111 glass fiber filter paper, which has a nominal thickness of 0.055 inches and is commercially available from Hollingsworth and Vose Co., East Walpole, Massachusetts. The thickness of such a material is not critical, and is a matter of choice for one skilled in the art, largely based upon the properties of the sample being assayed, such as its fluidity.

The microparticles employed to perform the solid-phase immunoassay are selected to have an average diameter smaller than the average spatial separation of the fibers that form the fibrous matrix. Also, the size of the microparticles employed is preferably small enough such that they are suspendable in water or sucrose to facilitate their coating with an antibody or antigen. The average individual size of the microparticles which can meet both of the above requirements is from about 0.1 to about 10 microns, more preferably from about 0.1 to about 5 microns in diameter. The microparticles can be selected from any suitable type of particulate material such as polystyrene, polymethylacrylate, polypropylene, latex, polytetrafluoroethylene, polyacrylonitrile, polycarbonate or similar materials.

Uncoated microparticles can be employed for binding some analytes but in many situations the particles are coated with a substance for binding an analyte, e.g., antibody or antigen, or a combination thereof.

The fibrous matrix 22 is positioned against the holding means 20 and above an absorbent material 24. The absorbent material 24 is generally cylindrical and functions to facilitate the transportation of fluid through the fibrous matrix 22. The absorbent material 24 can be any moisture or fluid-retaining material which effectively retains fluid passing through the fibrous matrix 22. Generally the absorbent material 24 can be composed of natural or synthetic fibers or other materials capable of absorbing fluid, i.e. cotton, cellulose acetate glass fibers, and synthetic or natural sponge. Preferably an absorbent material is employed where the fibers run perpendicular to the lower surface of the fibrous matrix to provide a capillary action to assist in drawing fluid through the fibrous matrix.

The absorbent material 24 is generally positioned below the fibrous matrix 22 to absorb any reagent or sample that flows through the fibrous matrix 22. One preferred manner for positioning the absorbent material 24 is to place it in intimate contact with the lower surface of the porous, fibrous matrix 22. This contact assures rapid transportation of the reaction fluids through the fibrous matrix 22.

The absorbent material 24 can be surrounded by a paper or cellophane wrapper 26 as shown in Fig. 2. The primary function of the wrapper 26 is to facilitate installation of the absorbent material into the reaction well 18 and prevent damage to the absorbent material 24 itself. The reaction well can include molded features to properly position the absorbent material 24 into the reaction well 18.

As an example, molded fins extending from the interior side walls of the reaction well 18 which are wider at their base than at their tops (not depicted in Figs. 1 or 2), can be employed. The fins would effectively force the absorbent material 24 up into the fibrous matrix 22 which in turn firmly positions the fibrous matrix 22 against the holding means 20. More preferably and as illustrated in Figure 2 a molded spike 28 is located at the base of the reaction well 18. The spike 28 forces the absorbent material 24 into intimate contact with the lower surface of the fibrous matrix 22.

The transfer and treatment of a sample with reagents in the cartridge is preferably but not necessarily accomplished by robotic means. Robotic arms can supply the necessary reagents by various transferring means communicating with reagent containers located external to the cartridge and associated carousel. The robotic arms can also aspirate and transfer the sample or reaction products to any of the respective wells.

In an automated solid-phase assay procedure the robotics are controlled by associated electronic and computer hardware and software designed to perform predetermined steps for a particular assay. In a preferred embodiment the necessary hardware, software and robotics system is based upon an adaptation of the TDx® instrument (a registered trademark of Abbott Laboratories, N. Chicago, Illinois) which is a commercially available automated instrument system originally designed for monitoring therapeutic drug concentrations in serum or plasma based on a method of fluorescence polarization immunoassay. The TDx® instrument is described in greater detail in an article by Alpert, Nelson L., Clinical Instrument System, Vol. 3, No. 11 (November 1982), herein, incorporated by reference.

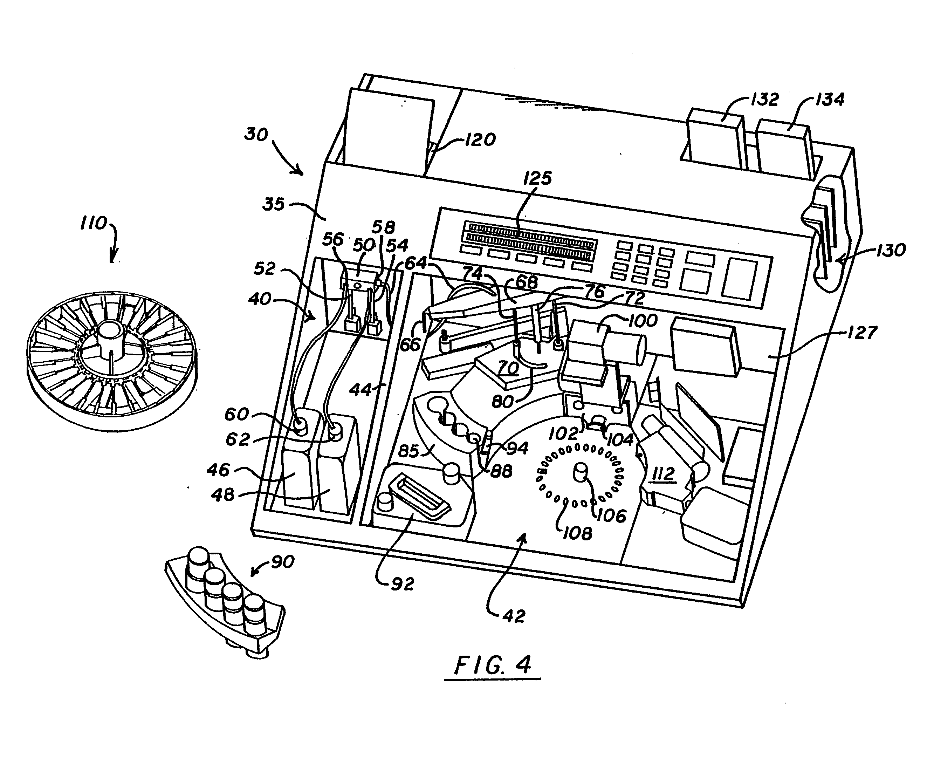

This preferred embodiment is illustrated by Fig. 4 where an automated analyzer 30 useful for performing an automated assay with the described cartridge is shown. The automated analyzer 30 comprises a number of individual mechanical, electrical, and optical components all mounted in a housing 35 also referred to as a "skin". The skin is divided into two sections 40 and 42 divided by a wall 44. A pair of buffer bottles 46 and 48 each containing a relatively large supply of a rinse or buffer solution are seated in an upright position in section 40. A dual pump block 50 controls the upward and downward travel of a pair of syringes 52 and 54 which are connected thereto. A corresponding pair of input ports 56 and 58 are connected by hollow tubing through the tops 60 and 62 of the buffer bottles 46 and 48 to the buffer solution contained therein. An output port of the pump block 50 is connected by a length of hollow tubing 64 across the dividing wall 44 to a pipetting means 66.

The pipetting means 66 is mounted on a mechanical robotic boom arm 68 which is in turn mounted by three vertical support rods 72, 74, and 76 to drive means (not shown) inside a base 70. Support rod 72 is connected and is rotationally driven by a conventional stepper motor (not shown) mounted in the base 70. A slot 80 is provided in the base 70 which allows the shaft 74 to move freely in a generally radial direction within certain limits about the axis of the rod 72 as it is rotationally driven by its stepper motor. As the support rod 72 is rotationally driven, the rod 76 rotates in place about its center axis. Thus, the rod 72 and associated driver motor provide the boom arm 68 with the ability to move laterally within certain limits. The rod 76 is machined such that the lower portion thereof is in the form of a worm lead. the lower portion of the rod 76 mates in conventional fashion with a gear and stepper motor (not shown) mounted in the base 70. The motor drives the gear which in turn drives the rod 76 vertically by way of the worm lead portion thereof to provide the boom arm with the ability to move vertically, within limits.

The pipetting means 66 is mounted vertically to the extreme end of the boom arm 68 by suitable mechanical connectors. As described above, the pipetting means is connected to the pump block by a length of hollow tubing. The pump block controls the operation of the pipetting means which is operative to selectably aspirate or expel selected volumes of fluids in a manner known to those skilled in the art. A reagent block 85 is mounted in section 42 adjacent to the base 70. The reagent block contains a plurality of wells 88 which are employed to seat a corresponding plurality of reagent containers 90 which in the preferred embodiment are configured in an integrated reagent pack. The wells 88 are arranged in a generally radial configuration directly beneath the pipetting means 66. Thus, as the boom arm 68 moves radially, the pipetting means traverses a radial path directly over each of the wells. Thus, when reagent containers 90 are seated in the block, the pipetting means can be positioned to selectively access each container.

Mounted in proximity to the reagent block is a waste bottle 92. The waste bottle 92 is connected to a rinsing fixture 94 which is mounted to the reagent block in radial alignment with the wells 88 so that it also is accessible by the pipetting means. The rinsing fixture 94 provides a fixture for the pipetting means to expel rinse or buffer solution during a rinse or wash step of an assay. The waste bottle provides a receptacle for the contaminated rinse solution which drains into the bottle from the rinsing receptacle. When the bottle is full, it can be removed from the analyzer and emptied, then replaced in the analyzer to receive more waste solution.

An optical reader assembly 100 is also mounted adjacent to the base 70. A motor 102 and pinion gear 104 driven thereby are mounted below the optical assembly with the pinion gear extending through the base of the skin 35. A support post 106 and air holes 108 which are machined in the base of the skin 35 radially around the support post 106 are located in front of the optical assembly and motor and pinion gear 104 with sufficient clearance to allow a carousel 110 to be seated on the support post. The carousel 110 is preferably of the type adapted to hold a plurality of the disposable diagnostic cartridges. Such a carousel is described in detail in a co-pending application entitled "Locking Rack and Disposable Sample Cartridge" filed concurrently, herewith, in the name of Cass Grandone and assigned to Abbott Laboratories, North Chicago, Illinois. The carousel has on its bottom surface a plurality of high resolution drive slots radially arranged along the outer periphery of the carousel. These slots mate with the teeth of the pinion gear 104 such that the carousel can be selectively rotationally driven in an indexed or stepwise fashion by the stepper motor 102.

An optical reader 112 is mounted adjacent to the motor and pinion gear and arranged generally radially therefrom to provide clearance for the carousel 110. The optical reader 112 is of conventional design and is provided to read a home position of the carousel in order to ensure that at the beginning and end of each test protocol, the carousel is in a known position. The carousel is provided with a reflective tab mounted thereon at the level of the optical reader for this purpose. As the carousel 110 rotates, the optical reader continuously outputs a signal indicative of whether a reflection has been detected. When a reflection is detected, the generated signal indicates to the analyzer that the carousel is in its home position and a test can begin. When the carousel returns to its home position, the reflection-induced signal indicates to the analyzer that a test of the entire batch of samples on the carousel 110 has been completed.

A conventional thermal printer 120 is controlled by the analyzer to provide a record of the results of the tests as well as other information that can be selected by the operator. A display 125 is provided on the skin 35 where it can be easily viewed by an operator of the analyzer. The display is of conventional design and is controlled in a conventional fashion by the analyzer 30 to provide various information to the operator such as the identity of the assay currently programmed, the status of any assay being conducted, and the results of diagnostic self-tests performed by the analyzer on its own internal system and components. A conventional keypad and touch switches are also provided on the skin for entering instructions to cause the analyzer to execute a diagnostic self test or perform other pre-programmed functions.

A back wall 127 divides the section 42 from an electronics section 130. The electronics section contains the control and processing electronics which control the operation of the analyzer and process the signals from the optical reader assembly to read the assays. The control and processing electronics are mounted on conventional printed circuit boards. The electronics section can include electrical connections that provide an interface to pre-programmed memory cartridges 132, and 134 which contain pre-programmed instructions for controlling the system and for causing the analyzer to execute the operations necessary to perform a specific assay or assays respectively. By changing assay cartridges 134, the analyzer can be selectively programmed to perform any number of known assays.

In operation, a laboratory technician or other operator loads the carousel 110 manually with a plurality of disposable cartridges containing samples to be assayed. The operator also selects a reagent pack containing the specific reagents for the assay to be performed and seats the reagent pack in the reagent block. The operator then mounts the loaded carousel 110 into the analyzer 30 and positions it on the support rod 106. Next the operator selects the appropriate pre-programmed assay cartridge and inserts the cartridge and a system cartridge in the two edge connectors provided therefore. The system may then be programmed for automatic operator not requiring any further operator intervention, or may require the operator to depress the RUN button on the skin 35 of the analyzer 30 to initiate the assay as desired.

The analyzer is capable of performing any number of different pre-programmed protocols and it is impossible to describe all of the available protocols herein. Instead, the sequence of operation of the analyzer is disclosed below in a manner generic to the cartridge. The programmed microprocessor controls the sequence and timing of the addition of reagents and sample to form a reaction mixture according to the pre-programmed assay protocol. After the reaction mixture has been deposited on the fibrous matrix, the microprocessor issues control signals to index the carousel until the next sample on the carousel is positioned in line with the reagent containers. The assay preparation steps are then repeated for this sample, and so on.

At the same time the microprocessor is controlling the preparation of assay reaction mixtures at the preparation station, it controls the optical reading of completed assays at the reading station. The optical reading apparatus has an optical source and an optical receiving means both of which overhand the carousel 110 and both of which are aligned with the reaction wells of the cartridges mounted on the carousel. When a cartridge is indexed into position beneath the reading assembly, the microprocessor issues control signals to cause a rate reading of the assay to be made. The microprocessor issues control signals to the optical assembly that cause the optical source to provide light pulses at a selected wavelength and at selected time intervals to illuminate the top surface of the fibrous matrix across the reaction well of the cartridge. Each time a light pulse at the selected wavelength illuminates the fibrous matrix, a fluophor-label bound to the analyte being assayed and trapped on or near the surface of the matrix fluorescence and emits light at a second wavelength. The intensity of this light indicates the concentration of the analyte in the original sample as well known to these skilled in the art. Each time a light pulse is emitted by the optical source, the optical receiver detects the intensity of the light emitted from the fibrous matrix and provides a signal to the microprocessor indicative of the intensity. The microprocessor is programmed according to a rate program known to those skilled in the art to determine the concentration of the analyte in the sample from the rate information provided by the optical assembly. Although rate readings are preferred, it is understood that the microprocessor can be easily programmed to take other types of readings as well.

Each time a sample preparation and an assay reading step are completed, the microprocessor is programmed to index the carousel to bring the next set of cartridges on the carousel 110 into preparation and reading position. Before issuing control signals for an assay preparation or reading procedure, the microprocessor reads the output of the carousel home position optical reader to determine whether the carousel is back in its home position. If the optical reader indicates that the carousel is in the home position, the microprocessor knows that it has completed the testing of an entire batch of samples and stops. The microprocessor may also be programmed if desired to display a message for the operator on the display or the thermal printer, and to output the results of the assays on the display or thermal printer is desired.

Methods for performing a solid-phase immunoassay with the cartridge require the use of microparticles which complex with the analyte sought to be assayed. Whether the solid-phase assay performed is a sandwich assay or a competitive assay the results of the test are dependent upon the ability of the microparticles to become retained and immobilized on the fibrous matrix.

For illustration purposes the following procedures are provided:

In one form of a sandwich assay method a sample is added to one of the empty wells, preferably well 12 as illustrated in Figs. 1 and 2, and the cartridge is placed in a carousel designed to hold a plurality of cartridges. Steps (a) through (e) may be performed by a microprocessor-controlled automated instrument or manually as follows:

(a) an analyte-specific conjugate is added a well containing a sample to form a mixture which is incubated for a sufficient time to allow any analyte present to complex with the analyte specific conjugate;

(b) the mixture is transferred to another empty well where microparticles are added to form a microparticle, analyte/conjugate complex; alternatively, the analyte/conjugate complex can be transferred to the reaction well to which microparticles have been previously or simultaneously added;

(c) the incubated microparticle, analyte/conjugate complex is transferred to the receiving port of the reaction well and washed with a suitable buffer or water to transport the complex into the porous fibrous matrix;

(d) an indicator substance capable of producing a color change or other detectable signal in the presence of the microparticle analyte/conjugate complex is added to the reaction well; and

(e) the assay signal is detected by optical means as a function of the presence or amount of analyte in the sample.

In a variation of the above procedure, steps (a) and (b), i.e., formation of analyte/conjugate complex and formation of microparticle, analyte/conjugate complex, respectively, can be performed simultaneously by adding the microparticles, sample and analyte-specific conjugate to an empty well and incubated. The complex is then transferred to the reaction well.

In the final step (e), detection of the assay signal can be affected by a carousel transporting the cartridge into alignment with an optics system to read an assay signal formed on the fibrous matrix of the reaction well. For example, where the assay signal is fluorescence the optics system will detect a fluorescence intensity reading from the porous fibrous matrix as a function of the presence or amount of analyte in the sample. This particular method of reading fluorescence intensity is called front-face fluorescence intensity reading.

Alternatively, step (e) can be performed by a series of rapid readings to determine the rate of formation of the complex consisting of the indicator substance (substrate) and the microparticle analyte/conjugate complex. The results obtained can then be graphed against a standard to determine the presence and concentration of analyte . This method of rapidly reading the generation of color intensity, or fluorescence at the reaction well provides the advantages of expanded assay range, improved assay sensitivity, greater signal to noise ratio and an automatic background subtraction built into the rate measurement.

In another version of a solid-phase sandwich assay procedure the automated or manual steps can be performed as follows:

(a) a sample and microparticles are mixed together either in the reaction well or another well to form a complex of the microparticle and analyte (if the complex is formed by incubating in a separate well then it is transferred to the reaction well);

(b) the microparticle analyte complex is treated with an analyte specific conjugate and incubated to form a microparticle, analyte/conjugate complex (alternatively steps (a) and (b) can be performed simultaneously by adding sample, microparticles and an analyte specific conjugate to a well, incubating and transferring the complex formed to the reaction well);

(c) the complex is then transported into the fibrous matrix by applying a wash of a suitable buffer or water;

(d) an indicator substance capable of producing a signal in the presence of the microparticle analyte/conjugate complex is added to the reaction well to form a assay signal;

(e) the assay signal is detected by optical means as a function of the presence or amount of analyte in the sample.

In yet another solid-phase immunoassay approach the disposable cartridge can be employed to perform a competitive binding assay. The automated or manual steps are as follows:

(a) a sample is added to a known amount of labeled antigen and microparticles capable of binding a suspect antigen to form a mixture;

(b) the mixture is transferred to the reaction matrix where the microparticles become bound to the fibrous matrix;

(c) the fibrous matrix is washed to remove unbound antigen;

(d) an indicator substance is added to the reaction well to form an assay signal in the presence of the labeled antigen; and

(e) the assay signal is detected by optical means as a function of the presence or amount of analyte in the sample.

It should be apparent that many variations of the above steps can be designed to form the microparticle, analyte/conjugate, substrate complex which can be detected by optical means on the porous, fibrous matrix. Generally the sandwich or competitive assay procedure and the choice of an analyte specific conjugate, and indicator substance are known to those skilled in the art and therefore are not discussed in great detail here. Instead, the present invention is directed toward the cartridge described above and in a preferred embodiment is suitable for the automated performance of a solid-phase immunoassay process on a microprocessor-controlled automated instrument.

Following are examples of solid-phase assays performed with the subject cartridge. In the examples the cartridge was used on a modified TDx® analyzer reprogrammed to perform the steps as outlined below and whose optics system was modified to read a fluorescent assay result from the surface of the reaction well, i.e., a front-face fluorescence intensity reading.

Example 1

A disposable cartridge having four wells as generally depicted in Figs. 1 and 2 was employed. The outermost well 18 is a reaction well and the innermost well 12 is a sample well. To the innermost well, the sample well, was added 25 microliters of specimen and the cartridge was placed in a carousel. Two automated assays for carcinoembryonic antigen (CEA) were then performed by employing protocols A and B as indicated below. The conjugate was an antibody-enzyme conjugate prepared from CEA specific antibody and horse-radish peroxidase.

Protocol A

a) the fibrous matrix of the reaction well was prewet by pipetting 75 microliters of buffer into the entrance port;

b) 25 microliters of microparticles having carcinoembryonic antigen specific antibody coated thereon in a buffered solution were added to the reaction well with 25 microliters of sample transferred from the sample well and the total mixture was allowed to incubate for 10 minutes at 35°C;

c) 25 microliters of enzyme conjugate (prepared from horseradish peroxidase phosphatase) were added to the reaction well and incubated for 10 minutes at 35°C;

d) 87 microliters of a wash solution (0.1% Tween-20) were added twice to the reaction well;

e) 100 microliters of substrate (3,3',5,5'-tetramethyl benzidine) were added to the reaction well and incubated for 10 minutes;

f) the color intensity of the reaction matrix was read.

Protocol B

a) 25 microliters of specimen transferred from the sample well and 100 microliters of conjugate (as in step (c) above) was mixed and incubated in a separate well for 10 minutes at 35°C;

b) 75 microliters of buffer were added to the reaction well;

c) 25 microliters of microparticles (as in step (b) above) were added to the reaction well, and 100 microliters of the specimen conjugate mixture were transferred to the reaction well and allowed to incubate for 10 minutes at 35°C; steps (d), (e) and (f) were performed as in Protocol A, above.

The color intensity was measured by reflectance off the surface of the fibrous matrix and reported as a ratio of 633 to 492 nm color intensity. The results are based on the quantity of CEA introduced into each specimen tested and are shown in Table I. The results indicate the successful performance of a solid-phase assay which has sensitivity sufficient for diagnostic purposes.

## Table I.

| CEA Concentration (ng/ml) | Colorimetric Reflectance | |
|---|---|---|
| | Protocol A | Protocol B |
| 0 | 0.750 | 0.747 |
| 1 | 0.712 | 0.744 |
| 4 | 0.641 | 0.719 |
| 10 | 0.595 | 0.641 |
| 28 | 0.403 | 0.493 |
| 60 | 0.320 | 0.387 |

Example 2

A disposable cartridge as employed in Example I was employed to perform an automated hCG assay. The microparticles employed were 0.5 microns in diameter and had a coating of $\beta$-hCG specific antibody. The conjugate was an antibody-enzyme conjugate prepared from hCG specific antibody and alkaline phosphatase. Approximately 50 microliters of specimen were placed in the sample well and the cartridge was placed into a carousel. An automated assay was run for hCG using the following protocol.

a) 25 microliters of antibody-enzyme conjugate was added to the an empty well and incubated with transferred sample for 10 minutes at 37°C;

b) 50 microliters of microparticles were added to the same well of step (a) and incubated for 10 minutes at 37°C;

c) 125 microliters of the sample well mixture were transferred to the reaction well and washed with 150 microliters of diluent;

d) 150 microliters of a wash solution (0.1% Tween 20) were added to the reaction well;

e) 50 microliters of enzyme substrate were added to the reaction well and incubated for 5 minutes;

f) percent reflectance of fluorescence was measured.

The fluorescence was determined by measuring the front-surface fluorescence of the surface of the fibrous matrix of the reaction well. The results as shown in Table II indicate the successful performance of a fluorometric solid-phase assay.

## Table II

| hCG Concentration (mIU/ml) | Fluorescence (Millivolts) |
|---|---|
| 0 | 0.59 |
| 5 | 0.69 |
| 10 | 0.83 |
| 50 | 1.74 |
| 100 | 3.07 |
| 200 | 5.70 |
| 500 | 9.30 |
| 1000 | 11.08 |

### Example III

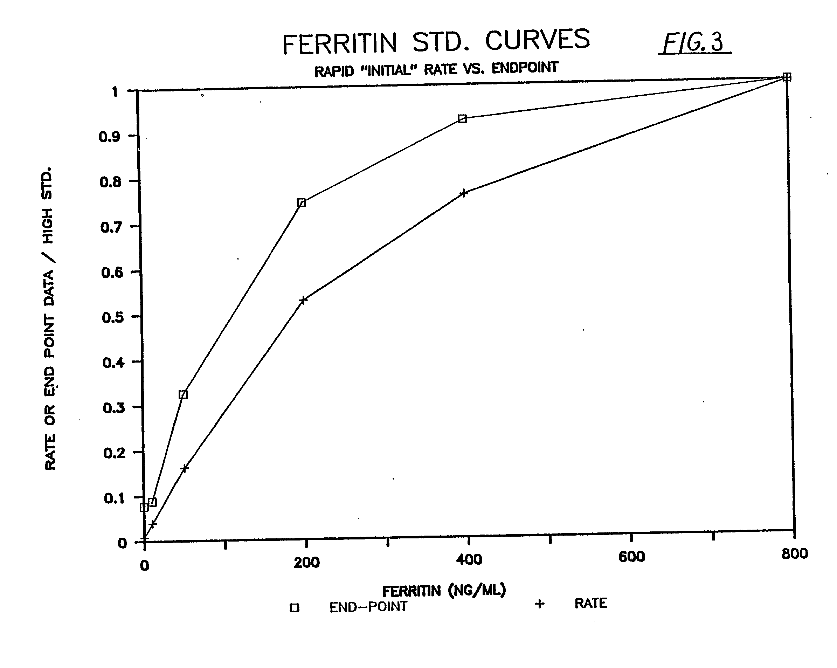

A disposable cartridge as employed in Examples I and II was employed to perform an automated assay protocol for ferritin. In this protocol multiple readings were taken to determine the generation of fluorescence at the reaction well. By taking rapid, successive readings of the fibrous matrix a curve could be generated which indicated the generation of fluorescence as is shown in Figure 3.

The specimens employed were ferritin standards prepared in stripped normal human serum. The reagents included an alkaline phosphatase conjugate diluted in a buffer and a flourescent substrate. The microparticles were coated with anti-ferritin specific mouse monoclonal antibody.

Approximately 45 microliters of specimen was placed in the sample well and the cartridge was placed into the carousel. The automated assay protocol was then performed as follows:

a) the specimen was transferred to another well where it was treated with 50 microliters of microparticles, 25 microliters of conjugate, 25 microliters of surfactant and 75 microliters of diluent and incubated for 15 minutes at 37°C;

b) 95 microliters of the mixture were then transferred to the reaction well and the mixture washed two times with 75 microliters of diluent;

c) 40 microliters of substrate were then added to the reaction well;

d) the cartridge was then advanced to the optics system and the fluorescence read seven seconds after addition of substrate for 250 milliseconds, delayed 1 second, then read for another 250 milliseconds. This delay and read sequence was repeated four times.

The rapid readings were used to construct a rate of substrate conversion which is graphed as time in seconds versus fluorescence counts. This data was then used to compare a ferritin standard and a specimen to calculate the ferritin concentration in the specimen. A graph of the rapid rate readings for the automated ferritin assay appears as Figure 3. The initial reading and end point readings are shown in Table III. The rapid rate method of reading the automated assay provides greatly enhanced accuracy and sensitivity in determining an analyte.

## Table III

| Ferritin Concentration (ng/ml) | Average Fluorescence | |
|---|---|---|
| | Initial Rate (Counts/Sec.) | End-Point (Counts) |
| 0 | 5 | 1536 |
| 10 | 21 | 1761 |
| 50 | 82 | 6475 |
| 200 | 267 | 14805 |
| 400 | 383 | 18345 |
| 800 | 505 | 19911 |

## Claims

1. A cartridge suitable for performing an automated solid-phase diagnostic assay which comprises a plurality of separate wells arranged in a predetermined order wherein at least one of said wells is a sample well for receiving a sample material to be assayed and at least one of said wells is a reaction well comprising:

(a) an entrance port and holding means for receiving and holding a quantity of sample and assay reagents;

(b) a fibrous matrix for retaining and immobilizing microparticle/analyte complexes for detection, said fibrous matrix positioned below said holding means, and having an average spatial separation of fibers greater than the average diameter of said microparticles; and

(c) an absorbent material positioned below said fibrous matrix to absorb any sample or assay reagents that flow through said fibrous matrix.

2. The cartridge of claim 1, wherein said fibrous matrix has an average spatial separation of fibers greater than 10 microns.

3. The cartridge of claim 1, wherein said absorbent material is made from fibrous material predominantly lying in a plane perpendicular to said lower surface of said fibrous matrix.

4. The cartridge of claim 1, wherein said reaction chamber includes positioning means for securely holding said absorbent material in contact with said fibrous matrix.

5. A method of performing a solid-phase diagnostic assay in a cartridge having a plurality of separate wells at least one of said wells being a sample well for receiving a sample to be assayed and another being a reaction well having a fibrous matrix for retaining and immobilizing microparticle analyte complexes and an absorbent material positioned below said fibrous matrix; the steps of said method comprising:

(a) incubating a sample with an analyte specific conjugate in one of said wells other than said reaction well to form an analyte/conjugate complex;

(b) adding said analyte/conjugate complex to said reaction well in the presence of microparticles having an average diameter smaller than the average spatial separation of the fibers forming said fibrous matrix to form a microparticle, analyte/conjugate complex;

(c) adding a wash solution to said reaction well to transfer said microparticle, analyte/conjugate complex into said fibrous matrix where it is retained and immobilized;

d) adding to said reaction well an indicator substance capable of forming an assay signal in the presence of said microparticle, analyte/conjugate complex; and

(e) detecting said assay signal produced by said indicator substance in step (d) as a function of the presence or amount of analyte in said sample.

6. The method of claim 5, wherein step (b) is performed by first contacting said analyte/conjugate complex with said microparticles to form a microparticle, analyte/conjugate complex and then transferring said microparticle, analyte/conjugate complex to said reaction well.

7. The method of claim 5, wherein steps (a) and (b) are performed simultaneously incubating a sample, microparticles and an analyte specific conjugate in one of said wells other than said reaction wells and then the microparticle, analyte/conjugate complex is transferred to said reaction well.

8. The method of claim 5, wherein step (e) consists of performing a series of rapid readings to determine a rate for the complexing of said indicator substance with said microparticle, analyte/conjugate complex.

9. A method of performing a solid-phase diagnostic assay in a cartridge having a plurality of separate wells at least one of said wells being a sample well for receiving a sample to be assayed and another being a reaction well containing a fibrous matrix for retaining and immobilizing microparticle, analyte complexes and an absorbent material positioned below said fibrous matrix; the steps of said method comprising:

(a) adding a sample to said reaction well in the presence of microparticles having an average diameter smaller than the average spatial separation of the fibers forming said fibrous matrix, and incubating to form a microparticle, analyte complex;

(b) adding to said reaction well and thereby to said microparticle, analyte complex an analyte specific conjugate and incubating the mixture to form a microparticle analyte/conjugate complex;

(c) adding a wash solution to said reaction well to transfer said microparticle, analyte/conjugate complex into said fibrous matrix where it is retained and immobilized;

(d) adding to said reaction well an indicator substance capable of forming an assay signal in the presence of said microparticle, analyte/conjugate complex; and

(e) detecting said assay signal produced by said indicator substance in step (d) as a function of the presence or amount of analyte in said sample.

10. The method of claim 9, wherein step (a) is performed by incubating said sample with said microparticles to form a microparticle, analyte complex whereafter said microparticle, analyte complex is transferred to said reaction well.

11. The method of claim 9, wherein step (b) is conducted by incubating said microparticle, analyte complex and an analyte specific conjugate to form a microparticle, analyte/conjugate complex which is transferred to said reaction well.

12. The method of claim 9, wherein said step (e) consists of performing a series of rapid readings to determine a rate for the complexing of said indicator substance with said microparticle, analyte/conjugate complex.

13. A method of performing a solid-phase diagnostic assay in a cartridge having a plurality of separate wells at least one of said wells being a sample well for receiving a sample to be assayed and another being a reaction well containing a fibrous matrix for retaining and immobilizing microparticle, analyte complexes and an absorbent material positioned below said fibrous matrix; the steps of said method comprising:

(a) treating a sample with a labeled antigen and microparticles capable of binding an antigen to form a reaction mixture thereof;

(b) transferring said reaction mixture to said reaction well whereby said microparticles become retained and immobilized on said fibrous matrix;

(c) washing said fibrous matrix to remove any unbound antigen;

(d) applying an indicator substance to said reaction well to form an assay signal with said labeled antigen;

(e) detecting said assay signal produced by said indicator substance in step (d) as a function of the presence or amount of antigen present in said sample.

13

FIG. 1

FIG. 2

FERRITIN STD. CURVES   *FIG.3*

RAPID "INITIAL" RATE VS. ENDPOINT

RATE OR END POINT DATA / HIGH STD.

FERRITIN (NG/ML)

□   END−POINT        +    RATE

0 288 793

FIG. 4